# EUROPEAN PATENT SPECIFICATION

(11) **EP 3 766 482 B1**
(45) Date of publication and mention of the grant of the patent: **22.11.2023**
(21) Application number: 20186404.8
(22) Date of filing: 17.07.2020
(51) Int. Cl.: A61K 9/127, A61K 9/51, A61K 31/00, A61K 47/69

(54) **DRUG DELIVERY BY PORE-MODIFIED MESOPOROUS SILICA NANOPARTICLES**
ARZNEIMITTELABGABE DURCH PORENMODIFIZIERTE MESOPORÖSE SILIZIUMNANOPARTIKEL
ADMINISTRATION DE MÉDICAMENTS PAR NANOPARTICULES DE SILICE MÉSOPOREUSE À PORES MODIFIÉS

(30) Priority: 18.07.2019 US 201962875822 P
(43) Date of publication of application: 20.01.2021
(73) Proprietor: Nano Targeting & Therapy Biopharma Inc., Taipei City 106 (TW); Chan, Hardy Wai Hong, New Taipei City 241 (TW)
(72) Inventor: CHAN, Hardy Wai Hong, 241 NEW TAIPEI CITY (TW); MOU, Chung-Yuan, 106 TAIPEI CITY (TW); WU, Cheng-Hsun, 302 HSINCHU COUNTY (TW); WU, Si-Han, 320 TAOYUAN CITY (TW); CHEN, Yi-Ping, 203 KEELUNG CITY (TW); ZHANG, Rong-Lin, 905 PINGTUNG COUNTY (TW)
(74) Representative: Novagraaf Technologies

(56) References cited:
- WO-A1-2014/165608
- WO-A1-2017/041032
- WO-A1-2018/160865
- WO-A2-2009/078924
- ALEXANDER LIBERMAN ET AL: "Synthesis and surface functionalization of silica nanoparticles for nanomedicine", SURFACE SCIENCE REPORTS., vol. 69, no. 2-3, 1 September 2014 (2014-09-01), pages 132-158, XP055523138, NL ISSN: 0167-5729, DOI: 10.1016/j.surfrep.2014.07.001
- Diti Desai ET AL: "Design considerations for mesoporous silica nanoparticulate systems in facilitating biomedical applications", Open Material Sciences, 1 January 2014 (2014-01-01), pages 16-43, XP055748704, DOI: 10.2478/mesbi-2014-0001 Retrieved from the Internet: URL:https://www.degruyter.com/view/journal s/oms/open-issue/article-10.2478-mesbi-201 4-0001/article-10.2478-mesbi-2014-0001.xml
- BOWEN P: "Particle Size Distribution Measurement from Millimeters to Nanometers and from Rods to Platelets", JOURNAL OF DISPERSION SCIENCE AND TECHNOLOGY, TAYLOR AND FRANCIS GROUP, NEW YORK, NY, US, vol. 23, no. 5, 1 January 2002 (2002-01-01), pages 631-662, XP009102859, ISSN: 0193-2691, DOI: 10.1081/DIS-120015368

## Description

### TECHNICAL FILED OF THE INVENTION

The present disclosure relates to mesoporous silica nanoparticles having modifications on the surface of the (extended) mesopores, which can be further loaded with one or more types of bioactive ingredients within the (extended) mesopores, processes of preparing the same and applications of the same.

### BACKGROUND OF THE INVENTION

Combination drug therapy is most widely used in treating the most dreadful diseases, such as cancer and infectious diseases. The major purposes of using drug combination are to achieve synergistic therapeutic effect, reduce dose and adverse effect, and minimize the induction of drug resistance. Hence, co-administration of two or more drugs to a patient usually shows greater therapeutic efficacy than each drug treatment alone. In cancer treatments, combination therapy seems to be a standard clinical practice to overcome drug resistance and enhance therapeutic outcome. The synergistic effect of two (or more) drugs with different mechanisms of action - which are targeting different cell-cycle checkpoints, genes, or metabolic pathways of cancer - raises the chances of eliminating cancer. However, the different physico-chemical and pharmacokinetic properties of drugs lead to many challenges when developing combination drug therapy; the main issues associated with the development of combination drug therapy are: (1) identification of appropriate drug combinations and drug ratios, (2) correlation of in vitro studies with behavior in vivo, and (3) whether the drugs can reach the same tumor cells in an effective dose and ratio (pharmacokinetic of drugs). Nanoparticle multidrug co-delivery provides a potential pathway to solve these challenges in combination drug therapy.

Nanoparticle formulations offer several advantages for multi drug co-delivery compared with combinations of free drugs such as: (1) nanoparticles can deliver hydrophobic and hydrophilic drugs at the same time and maintain the optimized synergistic drug ratio in a single nanoparticle, (2) nanoparticles can elongate circulation time and enhance the tumor targeting ability of drugs, and (3) drug encapsulated nanoparticles can simultaneously deliver multidrugs into target cells that will normalize the pharmacokinetic difference between the drugs. Mesoporous silica nanoparticles (MSNs) have been deemed to have great potential as drug delivery systems due to their unique physical/chemical properties, such as: large pore volume, chemical/thermal stability, high loading capacity, adjustable surface properties and excellent biocompatibility. The outer surface or pore surface of MSN can be easily modified with various functional groups individually. However, encapsulation of hydrophobic and hydrophilic drugs in the same particle usually affect the monodispersion of particles in solution, and therefore this delivery system still presents a challenge and is in need of improvement. In this invention, a small size MSN (<100 nm) with pore sizeexpanded (optionally)- and a pore surface modification by a specific functional group and ratio - can encapsulate two drugs (In one embodiment, one is hydrophobic, another is hydrophilic) in the same particle and show synergistic therapeutic effect in anti-multi drug resistant cancer cells. The multidrug codelivery by the particle mentioned in this invention can also be used for treating different diseases such as: cancers, multidrug resistant cancers, brain cancers, metastatic brain cancers, and central nervous system diseases.

WO2014165608 relates to antibiotic protocells comprising mesoporous nanoparticles encapsulated within a lipid bi- or multilayer.

WO2018160865 relates to mesoporous silica nanoparticles (MSNPs), monodisperse populations of MSNPs and related protocells which exhibit cell binding specificity.

WO2017041032 relates to mesoporous metal oxide nanoparticles and related protocells, and to the treatment and prevention of viral infections.

WO2009078924 relates to submicron structures including a silica body defining a plurality of pores that are suitable to receive molecules therein.

Other technological background documents may be cited as the article entitled *"synthesis and surface functionalization of silica nanoparticles for nanomedicine',* published by Alexander Liberman et al in Surface Science Reports, 2014, vol 69, N°2-3, pages 132-158; or the article entitled *"*Design consideration for mesoporous silica nanoparticulate system in facilitating biomedical applications', published in Open Material Science, 2014, , pages 16-43.

### Summary of the invention

The subj ect invention provides mesoporous silica nanoparticles having modifications on the surface of the mesopores. The mesoporous silica nanoparticles (MSN) have a pore size of smaller than 50 nm, and can be referred to as "exMSNs" when the pore size is greater than 3 nm (i.e., the pore is "extended").

The invention is set out in the appended set of claims.

The subject invention also provides pore-modified MSNs and exMSNs loaded with one or more types of bioactive ingredients within the (extended) mesopores. The subject application also provides methods of producing pore-modified MSNs and exMSNs with or without loading the bioactive ingredients.

In one aspect, the present disclosure provides a mesoporous silica nanoparticle, wherein the surface of the pores is modified with functional group(s) to stably trap or enclose one or more types of bioactive ingredients on the surface of the pores.

In one embodiment, the pore size of the mesoporous silica nanoparticle is smaller than 50 nm.

In one embodiment, the functional group is hydrophobic or hydrophilic or both.

In one embodiment, the surface of the pores of the mesoporous silica nanoparticle is modified with functional groups having a terminal hydrocarbyl moiety. In one embodiment, the terminal hydrocarbyl moiety comprises a terminal aromatic moiety, a terminal (cyclo)aliphatic moiety or combinations thereof.

In some embodiments, the terminal aromatic moiety is substituted with lower alkyl, or halogen. In a further embodiment, the terminal aromatic moiety is derived from trimethoxyphenylsilane (TMPS). In some embodiments, the terminal (cyclo)aliphatic moiety comprises (cyclo)alkyl, (cyclo)alkenyl or combinations thereof, which can be optionally substituted with lower alkyl or halogen.

In some embodiments, the bioactive ingredient is hydrophilic, hydrophobic or amiphiphilic.

In some embodiments, the bioactive ingredient is a small molecule, a chemo-drug, an enzyme, a protein drug, an antibody, a vaccine, an antibiotic or a nucleotide drug.

In some embodiments, at least two bioactive ingredients are loaded within the pores, e.g., onto the surface of the pores. In one embodiment, the mesoporous silica nanoparticle loads one or more hydrophilic bioactive ingredient and one or more hydrophobic bioactive ingredient within the pores, e.g., onto the surface of the pores. In one embodiment, the mesoporous silica nanoparticle loads one or more hydrophobic bioactive ingredients within the pores, e.g., on the surface of the pores.

In another aspect, the present disclosure provides a method for delivering bioactive ingredient(s) to a subject, comprising administrating the mesoporous silica nanoparticle of the present disclosure to the subject.

In one embodiment, the method can deliver mesoporous silica nanoparticle of the present disclosure to penetrate blood brain barrier and/or blood ocular barrier.

### Brief Description of the Drawings

Figure 1 shows the synergistic effect of JM17 and Dox co-delivery by exMSN on anti-MCF-7/ADR cancer cell.
Figure 2 shows the anti-MCF-7/ADR tumor efficacy of JM17 and Dox co-delivery by NTT2_131 nanoparticle.

### Detailed Description of the Invention

In order to facilitate the understanding of the disclosure herein, terms as used herein are hereby defined below.

In the context of the specification and the claims, the singular forms "a", "an" and "the" include plural referents, unless specifically indicated otherwise. Unless otherwise stated, any and all examples or exemplary language (e.g., "such as") provided herein are merely used for better illustration of the present invention, instead of limiting the scope of the present invention.

It is to be understood that any numerical range recited in this specification is intended to include all sub-ranges encompassed therein. For example, a range from "50 to 70°C" includes all sub-ranges and specific values between the stated minimum value of 50°C and the stated maximum value of 70°C, inclusive, e.g. from 58°C to 67°C, and from 53°C to 62°C, 60°C or 68°C. Since the numerical ranges disclosed are continuous, they contain each numerical value between the minimum and maximum value. Unless otherwise specified, the various numerical ranges indicated in this specification are approximate.

In the present invention, the term "about" refers to an acceptable deviation of a given value measured by a person of ordinary skill in the art, depending, in part, on how to measure or determine the value.

In the present invention, unless particularly specified, the prefix "nano-" as used herein means a size of about 300 nm or less. Unless particularly specified, the prefix "meso- " as used herein, means a size of no greater than about 50 nm.

In the present invention, the term "silane" as used herein refers to derivatives of SiH₄. Normally, at least one of the four hydrogens is replaced with substituents such as alkyl, alkoxyl, amino, etc. as described below. The term "alkoxysilane" as used herein refers to a silane having at least one alkoxyl substituent directly bonded to the silicon atom. The term "organo-alkoxysilane" as used herein refers to a silane having at least one alkoxyl substituent and at least one hydrocarbyl substituent directly bonded to the silicon atom. The term "silica source" as used herein refers to substances which can be deemed as a salt form or an ester form of orthosilicic acid, for example sodium orthosilicate, sodium metasilicate, tetraethyl orthosilicate (tetraethoxy silane, TEOS), tetramethyl orthosilicate, tetrapropyl orthosilicate. Optionally, the hydrocarbyl substituent can be further substituted or interrupted with a heteroatom.

In the present invention, the term "hydrocarbyl" as used herein refers to a monovalent radical derived from hydrocarbons. The term "hydrocarbon" as used herein refers to a molecule that consists of carbon and hydrogen atoms only. Examples of the hydrocarbons include, but are not limited to, (cyclo)alkanes, (cyclo)alkenes, alkadienes, aromatics, etc. When the hydrocarbyl is further substituted as mentioned above, the substituent can be halogens, amino groups, a hydroxy group, a thiol group, etc. When the hydrocarbyl is interrupted with a heteroatom as mentioned above, the heteroatom can be S, O or N. In the present invention, a hydrocarbyl preferably comprises 1 to 30 C atoms.

In the present invention, the term "alkyl" refers to a saturated, straight or branched alkyl, which comprises preferably 1-30 carbon atoms, and more preferably 1-20 carbon atoms. Examples of alkyl include, but are not limited to, methyl, ethyl, propyl, isopropyl, n-butyl, sec-butyl, isobutyl, tert-butyl, 2-ethylbutyl, n-pentyl, isopentyl, 1-methylpentyl, 1,3-dimethylbutyl, n-hexyl, 1-methylhexyl, n-heptyl, isoheptyl, 1,1,3,3-tetramethylbutyl, 1-methylheptyl, 3-methylheptyl, n-octyl, 2-ethylhexyl, 1,1,3-trimethylhexyl, 1,1,3,3-tetramethylpentyl, nonyl, decyl, undecyl, 1-methylundecyl, dodecyl, 1,1,3,3,5,5-hexamethylhexyl, tridecyl, tetradecyl, pentadecyl, hexadecyl, heptadecyl, octadecyl or the like.

In the present invention, the term "alkoxyl" or "alkoxy" as used herein means a group having a formula "-O-alkyl," wherein the definition of the "alkyl" in said formula has the meaning of "alkyl" as stated above.

In the present invention, the term "cycloalkyl" as used herein means a saturated or partially unsaturated cyclic carbon radical containing 3 to 10 ring carbon atoms and more preferably 3 to 8 ring carbon atoms, and optionally an alkyl substituent(s) on the ring. Examples of cycloalkyl include, but are not limited to, cyclopropyl, cyclopropenyl, cyclobutyl, cyclopentyl, cyclohexyl, 2-cyclohexen-1-yl, and the like.

In the present invention, the term "halogen" or "halo" denotes fluorine, chlorine, bromine or iodine.

In the present invention, the term "amino" as used herein means a functional group of the formula -NR₁R₂, wherein R₁ and R₂ each independently represent hydrogen or a hydrocarbyl group as defined above.

In the present invention, the term "aqueous phase" as used herein means a phase substantively miscible with water. Examples of the aqueous phase include, but are not limited to, water *per se,* aqueous buffers, aqueous dimethylsulfoxide (DMSO) solutions, aqueous alkanolic solutions, etc. The aqueous phase may be adjusted to be acidic, neutral or alkaline, based on the demand of the synthesis and/or the stability of the substance present in the aqueous phase.

In the present invention, the term "oil phase" as used herein means a phase substantively immiscible with the aqueous phase as mentioned above. Examples of the oil phase include, but are not limited to, liquid, substituted or unsubstituted (cyclo)alkanes, such as hexane, decane, octane, dodecane, cyclohexane, etc.; substituted or unsubstituted aromatic solvents, such as benzene, toluene, xylene, etc.

In the present invention, the term "bioactive ingredient" as used herein refers to substance having an activity in an organism. Examples of the bioactive ingredient include, but are not limited to, a small molecule, a chemo-drug, an enzyme, a protein drug, an antibody, a vaccine, an antibiotic or a nucleotide drug.

### Pore-modified MSNs

Nanoparticle formulations offer several advantages for multidrug co-delivery compared with combinations of free drugs such as (1). nanoparticle can deliver hydrophobic and hydrophilic drugs at the same time and maintain the optimized synergistic drug ratio in single nanoparticle, (2). nanoparticle can elongate circulation time and enhance tumor targeting ability of drugs, (3). Drug encapsulated nanoparticle can deliver multidrug into a target cell simultaneously that will normalize the pharmacokinetic difference between drugs. Mesoporous silica nanoparticles (MSNs) have been deemed to have great potential as drug delivery systems due to their unique physical/chemical properties, such as large pore volume, chemical/thermal stability, high loading capacity, adjustable surface properties and excellent biocompatibility. The outer surface or pore surface of MSN can be easily modified with various functional groups individually. However, encapsulation of hydrophobic and hydrophilic drug in the same particle did not affect the monodisperse of particle in solution is still a challenge and in need of improvement. In this invention, a small size MSN (<100 nm) with pore size-expanded (optionally) and pore surface modification by specific functional group and ratio can encapsulate two drugs (In one embodiment one is hydrophobic, another is hydrophilic) in the same particle and show synergistic therapeutic effect in anti-multi drug resistant cancer cell. The multidrug codelivery by the particle mentioned in this invention can also be used for treating different indications such as cancers, multidrug resistant cancers, brain-associated cancers, metastatic brain cancers, and central nervous system diseases.

The pore-modified MSNs and exMSNs of the invention have suitable apparent sizes, dynamic light scattering sizes and pore sizes such that they can sufficiently load bioactive ingredients and be transported in the circulation system of the subject. In certain embodiments, the pore-modified MSNs and exMSNs may even penetrate blood-brain barrier (BBB) and/or blood ocular barrier.

The pore size of the MSNs and exMSNs may affect the loading capacity and/or efficiency of bioactive ingredients. If the pore size is too small, the loading capacity of larger molecule may be insufficient. If the pore size is too large, the loading efficiency may be lower and the loaded drug may easily leak from pores. In one embodiment, the pore size of the MSNs and exMSNs is no greater than 50 nm preferably from 1 to 20 nm, more preferably from 3 to 10 nm, from 1 to 10 nm or from 1 to 5 nm. The pore size may be suitably controlled by proper synthetic procedure and/or materials based on the demand, e.g., based on the size of the bioactive ingredient to be loaded within the pores.

The sizes of the MSNs and exMSNs may play an important role in enhancing the transportation thereof in the circulation system. If the size is too large, the nanoparticles may be rapidly identified and cleared by the immune system. If the size is too small, the nanoparticles may be rapidly and easily cleared from the body by renal filtration. In one embodiment, the size of the MSNs and exMSNs is no greater than 100 nm, preferably no greater than 80 nm, no greater than 65 nm, no greater than 60 nm or no greater than 50 nm, more preferably no greater than 40 nm. In one embodiment, the size of the MSNs and exMSNs is at least 60 nm, preferably at least 40 nm, more preferably at least 30 nm. In one embodiment, the size of the MSNs and exMSNs ranges from a viable numeric range consisting of any points as disclosed herein as the endpoints of the numeric range, for example from 30 nm to 100 nm, etc.

The dynamic light scattering (DLS) size of the MSNs and exMSNs is used to evaluate the suspension in different solution. If the DLS size is too large, the particle may be easily aggregated in stock solution or under physiological condition, which may be disadvantageous in producing pharmaceutically stable compositions and providing a stable manufacture process; the compositions also may not be used in clinical applications due to the poor blood circulation and high risk of vascular obstruction. To better evaluate the potential of the MSNs and exMSNs for the applications in living subjects, the DLS size is preferably measured in both water and in a medium which is biologically similar to or equivalent to phosphate buffered saline (PBS). In one embodiment, the dynamic light scattering size of the MSNs and exMSNs is at least 60 nm, more preferably at least 30 nm. In one embodiment, the dynamic light scattering size of the MSNs and exMSNs is no more than 150 nm, preferably no more than 100 nm, more preferably no more than 60 nm or no more than 30 nm. In one embodiment, the dynamic light scattering size of the MSNs and exMSNs ranges from a viable numeric range consisting of any points as disclosed herein as the endpoints of the numeric range, for example from 30 nm to 150 nm, etc. Without being bound to the theory, nanoparticles having a DLS size over 150 nm may easily aggregate or be cleared by immune system and thus cannot properly deliver bioactive ingredients.

The surface of the pores of the MSNs and exMSNs is modified with functional group(s), hereinafter referred to as "internal surface modification." The internal surface modification may allow the bioactive ingredients to be more stably trapped or enclosed in the pores. The internal surface modification may also affect the dispersity of the MSNs and exMSNs. The functional group for the internal surface modification is normally hydrophobic, in particular aromatic. In one embodiment, the surface of the pores of the MSNs and exMSNs is modified with functional groups having a terminal hydrocarbyl moiety. In one embodiment, the hydrocarbyl moiety is an aromatic moiety selected from benzene (phenyl), naphthalene, anthracene, phenanthrene, diphenyl ether, ellagic acid, carbazolyl, quercetin, etc. In one embodiment, the aromatic moiety is substituted with lower alkyl, alkenyl, alkoxyl or halogen. In one embodiment, the aromatic moiety can be derived from aromatic siloxane, e.g., trimethoxyphenylsilane (TMPS). The amount (content, etc.) of terminal aromatic moiety can be measured to ensure the terminal aromatic moiety is sufficient. In one embodiment, the terminal hydrocarbyl moiety is a (cyclo)aliphatic moiety selected from (cyclo)pantanyl, (cyclo)hexanyl, (cyclo)heptanyl, (cyclo)octanyl, (cyclo)nonanyl, (cyclo)decanyl, (cyclo)undecanyl, (cyclo)dodecanyl, etc. In one embodiment, the terminal hydroconbyl moiety comprises an aromatic moiety, a (cyclo)aliphatic moiety or combinations thereof. Without being bound to the theory, when the amount of the terminal hydrocarbyl moiety on the internal surface is too high, the dispersity of the MSNs and exMSNs may be insufficient; when the amount (ratio) of the terminal moiety is too low, the loading capacity/efficient of hydrophobic bioactive ingredient may be insufficient.

The "outer" surface, i.e., surface of the particles, of MSNs and exMSNs can be modified with functional groups, which will also change the properties and thereby bioapplication performance of MSN. For example, poly(alkoxylene glycol) (PAG)-type group modification can make the particle exhibit better suspension in a medium, lower immunogenicity and longer circulation period in body. While MSNs without any outer surface modification, they normally bear negative charges on the surface. Hence, polyethylenimine (PEI), alkoxylsilane-terminated (poly)alkylene(poly)amine or aminecontaining organo-alkoxysilane modification can be applied to make the particles to have a positive or weak negative surface charge or to be electrically neutral on the outer surface. Carboxyl, phosphoryl, sulfonate-containing organo-alkoxysilane modifications on the other hand can make the particles to have strongly negative charges. In addition, combinations of functional groups on the surface of particles will provide multiple surface properties.

### EPR Effect of MSNs and exMSN

In general, EPR-mediated passive targeting highly relies on the prolonged circulation time of nanocarriers. The enhanced permeability and retention (EPR) effect based tumor targeting would be approached by (1) high-density PEGylation; (2) spatial control of functional groups on the surface; (3) making of small MSNs and exMSN and (4) controlling the protein corona formation. Particularly important two parameters are particle size and surface properties, which would be expected to play key roles on the circulation half-life, pharmacokinetics, and bio-distribution of the nanocarriers. Typically, the injected materials would be recognized and bound rapidly by serum opsonins, followed by phagocytosis and substantially accumulated in both the liver and the spleen (also known as the mononuclear phagocyte system). In addition, comprehensive studies highlighted protein corona neutrality as an important design in the development of targeted nanomaterial delivery and demonstrated that even a small difference in the surface heterogeneity could have chances to result in profoundly different interactions with cells and tissues. Therefore, the control and understanding of protein corona composition might be critical for developing successful EPR-targeted nanomedicines.

### BBB Penetration Effect

Blood-brain barrier (BBB) restricts most of therapeutic drugs transported into the brain. Nanomedicine can modulate the nanoparticle size, shape, surface charge, conjugated ligands to increase penetration of BBB. Nanoparticle conjugated with targeting ligands that bind to receptors on endothelial cells, such as transferrin, lactoferrin, glutathione and lowdensity lipoprotein receptors, may also promote BBB penetration. However, modification with targeting ligands on nanoparticle exterior surface may also affect the suspension and circulation of nanoparticles in blood and accelerate the blood clearance of nanoparticles. We based on varying and controlling the size, surface composition, and zeta potential of PEGylated MSN and exMSNs to increase the BBB penetration ability. Those modifications, spatial arrangements, and charges make MSNs and exMSNs reveal characteristics including minimal non-specific binding, proper circulation period in physiological environment and transport thereof from blood to brain

### Blood-Ocular Barrier Penetration Effect

The leading causes of vision impairment and blindness are posterior segment-related diseases including age-related macular degeneration, diabetic macular edema, glaucoma, endophthalmitis etc. However, blood-ocular barriers, like blood-brain barrier, refrain most of therapeutic drugs from being transported into the eye, especially to the posterior-segment of eye. For overcoming the barriers, the characteristics of MSN and exMSNs, such as the nanoparticle size, surface charge, and constitution, etc., can be modulated to increase penetration of static and dynamic barriers of eyes, thereby improving ocular bioavailability thereof. Therefore, the MSN and exMSNs will be a potential ocular drug delivery carrier for treating ocular diseases. The administration route of MSNs and exMSNs in such applications could be topical (eye drop), intravitreal, subconjunctival, subretinal, peribulbar, posterior juxtascleral, suprachoroidal retrobulbar, intracameral, sub-tenon, systemic injection, etc.

### Bioactive Ingredients

The bioactive ingredients used herein may be hydrophilic, hydrophobic or amiphiphilic. In one embodiment, the bioactive ingredient can be hydrophilic or being modified to be hydrophilic, and can be selected from those that are water soluble or that have surface modification making it capable of dispersing or dissolving in an aqueous phase. In one embodiment, the bioactive ingredient is an enzyme, a protein drug, an antibody, a vaccine, an antibiotic or a nucleotide drug. Examples of the enzyme include, but are not limited to, agalsidase, imiglucerase, taliglucerase, velaglucerase, alglucerase, sebelipase, laronidase, idursulfase, elosulfase, galsulfase, alglucosidase, asparaginase, glutaminase, arginine deiminase, arginase, methioninase, cysteinase, homocysteinase, phenylalanine hydroxylase, phenylalanine ammonia lyase, urate oxidase, catalase, horseradish peroxidase, superoxide dismutase or glutathione peroxidase.

In one embodiment, the bioactive ingredient may be properly selected based on the hydrophilic, hydrophobic or amiphiphilic thereof and the concerned disorders/diseases. Examples of the bioactive ingredient include, but are not limited to, everolimus, trabectedin, abraxane, TLK 286, AV-299, DN-I0l, pazopanib, GSK690693, RTA 744, ON 0910.Na, AZD 6244 (ARRY-142886),AMN-107, TKI-258, GSK461364, AZD 1152, enzastaurin, vandetanib, ARQ-197, MK-0457, MLN8054, PHA-739358, R-763, AT-9263, a FLT-3 inhibitor, a VEGFR inhibitor, an EGFR TK inhibitor, an aurora kinase inhibitor, a PIK-1 modulator, a Bcl-2 inhibitor, an HDAC inhibitor, a c-MET inhibitor, a PARP inhibitor, a Cdk inhibitor, an EGFR TK inhibitor, an IGFR-TK inhibitor, an anti-HGF antibody, a PI3 kinase inhibitors, an AKT inhibitor, a JAK/STAT inhibitor, a checkpoint-1 or 2 inhibitor, a focal adhesion kinase inhibitor, a Map kinase kinase (mek) inhibitor, a VEGF trap antibody, pemetrexed, erlotinib, dasatanib, nilotinib, decatanib, panitumumab, amrubicin, oregovomab, Lep-etu,nolatrexed, azd2171, batabulin, ofatumumab, zanolimumab, edotecarin, tetrandrine, rubitecan, tesmilifene,oblimersen, ticilimumab, ipilimumab,gossypol,Bio 111, 131-I-TM-601, ALT-110, BIO 140, CC 8490, cilengitide, gimatecan, IL 13-PE38QQR, INO 1001, IPdR1 KRX-0402, lucanthone, LY 317615, neuradiab, vitespan, Rta 744, Sdx 102, talampanel, atrasentan, Xr 311, romidepsin, ADS-I00380, sunitinib,5-fluorouracil, vorinostat, etoposide, gemcitabine, doxorubicin, liposomal doxorubicin, 5'-deoxy-5-fluorouridine, vincristine, temozolomide, ZK-304709, seliciclib; PD0325901 , AZD-6244, capecitabine, L-Glutamic acid, N -[ 4-[2-(2-amino-4,7 -dihydro-4-oxo-1-H-pyrrolo[2,3-d]pyrimidin-5-yl) ethyl]benzoyl]- disodium salt, heptahydrate, camptothecin, PEG-labeled irinotecan, tamoxifen, toremifene citrate, anastrazole, exemestane, letrozole, DES(diethylstilbestrol), estradiol, estrogen, conjugated estrogen, bevacizumab, IMC-1C11, CHIR-258, 3-[5-(methylsulfonyl piperadinemethyl)-indolyl]-quinolone, vatalanib, AG-013736, AVE-0005, goserelin acetate, leuprolide acetate, triptorelin pamoate, medroxyprogesterone acetate, hydroxyprogesterone caproate, megestrol acetate, raloxifene, bicalutamide, flutamide, nilutamide, megestrol acetate, CP-724714; TAK-165, HKI-272, erlotinib, lapatanib canertinib, ABX-EGF antibody, erbitux, EKB-569, PKI-166, GW-572016, Ionafarnib, BMS-214662, tipifamib; amifostine, NVP-LAQ824, suberoyl analide hydroxamic acid, valproic acid, trichostatin A, FK-228, SU11248, sorafenib, KRN951 , aminoglutethimide, amsacrine, anagrelide, L-asparaginase, Bacillus CalmetteGuerin (BCG) vaccine, bleomycin, buserelin, busulfan, carboplatin, carmustine, chlorambucil, cisplatin, cladribine, clodronate, cyproterone, cytarabine, dacarbazine,dactinomycin, daunorubicin, diethylstilbestrol, epirubicin, fludarabineetc. fludrocortisone, fluoxymesterone, flutamide, gemcitabine, hydroxyurea, idarubicin, ifosfamide, imatinib,leuprolide, levamisole, lomustine, mechlorethamine, melphalan, 6-mercaptopurine, mesna, methotrexate, mitomycin, mitotane, mitoxantrone, nilutamide, octreotide, oxaliplatin, pamidronate, pentostatin, plicamycin, porfimer, procarbazine, raltitrexed, rituximab, streptozocin, teniposide, testosterone,thalidomide, thioguanine, thiotepa, tretinoin, vindesine, 13-cis-retinoic acid, phenylalanine mustard, uracil mustard, estramustine, altretamine, floxuridine, 5-deooxyuridine, cytosine arabinoside, 6-mecaptopurine, deoxycoformycin, calcitriol, valrubicin, mithramycin, vinblastine,vinorelbine, topotecan, razoxin, marimastat, COL-3, neovastat, BMS-275291, squalamine, endostatin, SU5416, SU6668, EMD121974, interleukin-12, IM862, angiostatin, vitaxin, droloxifene, idoxyfene, spironolactone, finasteride,cimitidine, trastuzumab, denileukin diftitox, gefitinib, bortezimib, paclitaxel, cremophor-free paclitaxel, docetaxel, ixabepilone, epithilone B, BMS-247550, BMS-310705, droloxifene,4-hydroxytamoxifen, pipendoxifene,ERA-923, arzoxifene,fulvestrant, acolbifene, lasofoxifene, idoxifene, TSE-424, HMR-3339, ZK186619, topotecan, PTK787/ZK 222584, VX-745, PD 184352, rapamycin, 40-O-(2-hydroxyethyl)rapamycin, temsirolimus, AP-23573, RAD001, ABT-578, BC-210, LY294002, LY292223, LY292696, LY293684, LY293646, wortmarmin, ZM336372, L-779,450, PEG-filgrastim, darbepoetin, erythropoietin, granulocyte colony-stimulating factor, zolendronate, prednisone, cetuximab, granulocyte macrophage colony-stimulating factor, histrelin, pegylated interferon alfa-2a, interferon alfa-2a,pegylated interferon alfa-2b, interferon alfa-2b, azacitidine, PEG-L-Asparaginase, lenalidomide,gemtuzumab, hydrocortisone, interleukin-11, dexrazoxane, alemtuzumab, all-transretinoic acid, ketoconazole, interleukin-2, megestrol, immune globulin, nitrogen mustard, methylprednisolone, ibritgumomab tiuxetan, androgens, decitabine, hexamethylmelamine,bexarotene,tositumomab, arsenic trioxide, cortisone, editronate, mitotane, cyclosporine, liposomal daunorubicin, Edwina-asparaginase, strontium 89, casopitant, netupitant,an NK -1 receptor antagonists, palonosetron, aprepitant, diphenhydramine , hydroxyzine, metoclopramide, lorazepam, alprazolam, haloperidol, droperidol, dronabinol, dexamethasone, methylprednisolone, prochlorperazine, granisetron, ondansetron, dolasetron, tropisetron, pegfilgrastim, erythropoietin, epoetin alfa, curcumin, ALZ001, JM17 and darbepoetin alfa.

The bioactive ingredients may be in various forms such that they can be properly loaded into or onto the MSNs and exMSNs. Examples of the forms comprise, but are not limited to, an aqueous form, a dispersion form, an ionized form in a solution or a dispersion, a hydrated or solvated form in a solution or dispersion, etc.

### The MSNs and exMSNs Loaded with Bioactive Ingredients

The MSNs and exMSNs may load bioactive ingredients within the pores. Certainly, the subject invention can provide MSNs or exMSNs loading at least one bioactive ingredient. In particular, the subject invention provides MSNs or exMSNs loading at least two bioactive ingredients. The at least two bioactive ingredients may be similar or different in their hydrophobicity. In one embodiment, the MSNs or exMSNs load a hydrophilic bioactive ingredient and a hydrophobic bioactive ingredient. In one embodiment, the MSNs or exMSNs load at least two bioactive ingredients wherein at least one is hydrophilic and the rest is/are hydrophobic. In one embodiment, the MSNs or exMSNs load at least two bioactive ingredients wherein at least one is hydrophobic and the rest is/are hydrophilic. In one embodiment, the MSNs or exMSNs load at least two bioactive ingredients and all of them are hydrophilic. In one embodiment, the MSNs or exMSNs load at least two bioactive ingredients and all of them are hydrophobic. In one embodiment, the two or more bioactive ingredients provide synergistic effect.

The ratio of the bioactive ingredients can be adjusted to meet the requirements of intended purposes.

### Methods of Producing pore-modified MSN or exMSNs and nanoparticles Loaded with Bioactive Ingredient(s)

The subject application also provides methods of producing MSNs and exMSN with internal surface organic modification on the pores. In one embodiment, the method comprises the following steps:
(a) providing an alkaline solution containing a surfactant to form micelles;
(b) adding a first silica source and a second silica source which provides a terminal hydrocarbyl moiety into the solution, wherein the molar ratio of the first silica source and the second silica source is no less than 5: 1;
(c) conducting hydrothermal treatment to the solution; and
(d) extracting and optionally purifying the MSNs from the solution.

In a further embodiment, the method further comprises at least one of the following steps:
(e) introducing oil phase into the solution after step (a) and before step (b) for pore extension;
(f) adding a further silica source after step (b); and
(g) conducting surface modification of the external surface of the MSNs, wherein the surface modification is conducted after step (b), or after step (f) if step (f) is conducted.

The ratio of the first silica source to the second silica source which provides a terminal hydrocarbyl moiety can be adjusted such that the loading capacity and/or efficiency of bioactive ingredients of the MSNs thus prepared can be improved, particularly of no less than 5:1. In one embodiment, the molar ratio of the silica source and the terminal aromatic moiety providing reagent ranges from 29:1 to 4:1, preferably 26:1 to 5:1, more preferably 21:1 to 11:1. In addition, said ratio may be an important factor of affecting the DLS size of the MSNs and exMSNs. Without being bound to the theory, the applicant asserts that when the ratio of the first silica source to the second silica source is too low, the outer surface will bear organic modifications such that the DLS size will (dramatically) grow over 150 nm, which hampers the applications on the delivery of bioactive ingredients in living subject.

The modification agent for the internal surface modification can provide terminal hydrocarbyl moiety on the surface of the pores of the MSNs and exMSNs. Examples of the modification agent may include, but are not limited to, trimethoxyphenylsilane (TMPS), triethoxyphenylsilane, diphenyldiethoxysilane, 1-naphthyl trimethoxysilane, 2-hydroxy-4-(3-triethoxy silylpropoxy)diphenylketone, O-4-methylcoumarinyl-N-[3-(triethoxysilyl)propyl]carbamate, 7-triethoxysilylpropoxy-5-hydroxyflavone, 3-carbazolylpropyltriethoxysilane, bis(2-diphenylphosphinoethyl)methylsilylethyltriethoxysilane, 2-(diphenylphosphino)ethyl triethoxysilane, propyltriethoxysilane n-butyltriethoxysilane, pentyltriethoxysilane, hexyltriethoxysilane, heptyltriethoxysilane, octyltriethoxysilane, nonyltriethoxysilane, decyltriethoxysilane, undecyltriethoxysilane, dodecyltriethoxysilane, cyclopropyltriethoxysilane, cyclobutyltriethoxysilane, cyclopentyltriethoxysilane, cyclohexyltriethoxysilane, cycloheptyltriethoxysilane, cyclooctyltriethoxysilane, propyltrimethoxysilane n-butyltrimethoxysilane, pentyltrimethoxysilane, hexyltrimethoxysilane, heptyltrimethoxysilane, octyltrimethoxysilane, nonyltrimethoxysilane, decyltrimethoxysilane, undecyltrimethoxysilane, dodecyltrimethoxysilane, cyclopropyltrimethoxysilane, cyclobutyltrimethoxysilane, cyclopentyltrimethoxysilane, cyclohexyltrimethoxysilane, cycloheptyltrimethoxysilane, cyclooctyltrimethoxysilane etc.

Modifications of the external surface of the MSNs and exMSNs can be made *de novo* or can be post-modifications. Examples of the modification can be, but are not limited to, hydrophilic modifications, such as polyethylene glycol) (PEG) modification, polyethylenimine (PEI) modification, 3-(trihydroxysilyl)propyl methylphosphonate (THPMP) modification, N-(trimethoxysilylpropyl)ethylenediamine triacetic acid (EDTAS) N-[3-(trimethoxysilyl)propyl]ethylenediamine modification, N-[3-(trimethoxysilyl)propyl]-N,N,N-trimethylammonium (TA-trimethoxysilane) modification, (3-mercatopropyl)trimethoxysilane (MPTMS) modification, zwitterionic silane modification; specificity modifications, such as modifications with biomarkers, for example antibody modifications, linker modifications, tumor-targeting ligand modification, etc.; or non-specific activity modifications, such as modifications of surface properties of the shell, for example modification of the charge types or distribution, etc.

The subject invention also provides methods of producing MSNs and exMSNs loaded with bioactive ingredient(s). In one embodiment, the method comprises the following steps:
(a) providing MSNs or exMSNs as prepared in any of the above-mentioned methods;
(b) loading a first bioactive ingredient by contacting the MSNs or exMSNs with the first bioactive ingredient;
(c) loading a second bioactive ingredient by contacting the MSNs or exMSNs loaded with the first bioactive ingredient; and
(d) optionally, repeating step (c) for loading further bioactive ingredient(s) by independently and successively contacting the MSNs or exMSNs loaded with bioactive ingredients with the further bioactive ingredient(s).

In one embodiment, the first and second bioactive ingredients are similar or the same in hydrophobicity. In one embodiment, the first and second bioactive ingredients are different in hydrophobicity. In a specific embodiment, the first bioactive ingredient is hydrophilic and the second bioactive ingredient is hydrophobic. In another specific embodiment, the first bioactive ingredient is hydrophobic and the second bioactive ingredient is hydrophilic. In one embodiment, the further bioactive ingredient(s) are independently hydrophilic or hydrophobic.

### Applications of MSNs and exMSNs Loaded with Bioactive Ingredients

Mesoporous silica nanoparticles (MSNs) have been deemed to have great potential as drug delivery systems due to their unique physical/chemical properties, such as large pore volume, chemical/thermal stability, high loading capacity, adjustable surface properties and excellent biocompatibility. In particular, the MSNs and exMSNs of the subject application may have abilities of penetrating blood-brain barrier (BBB) and/or blood ocular barrier such that they can be used in specific applications.

### EXAMPLES

The following examples are provided to make the present invention more comprehensible to those of ordinary skill in the art to which the present invention pertains, but are not intended to limit the scope of the invention.

### Materials, Methodologies and Test Models

### Transmission Electron Microscopy (TEM)

Transmission electron microscopy (TEM) is used to directly examine and verify the appearance of the silica nanoparticles. The TEM images were taken on a Hitachi H-7100 transmission electron microscope operated at an accelerated voltage of 75-100 kV. Samples dispersed in ethanol were dropped on carbon-coated copper grids and air-dried for TEM observation.

### Dynamic Light Scattering (DLS)

Size measurements of the silica nanoparticles in different solution environments were performed with Dynamic Light Scattering (DLS) on a Malvern Zetasizer Nano ZS (Malvern, UK). The (solvated) particle sizes formed in different solutions were analyzed: H₂O, Dulbecco's Modified Eagle Medium (DMEM) with 10% FBS, PBS buffer solution (pH7.4) and 5% Glucose at room temperature.

### Elemental analysis

The mass percentage of carbon, nitrogen, oxygen and hydrogen in silica nanoparticle were determined by elemental analyzer (elementar Vario EL cube type for NCSH, German).

### Nanoparticle Tracking Analysis (NTA)

The particle concentration (number/mg) of sample solution is determined by Nanosight NS300.

### Qantification of Drugs in Nanoparticles

To 5 *µ*L of drugs-loaded nanoparticle dispersion (from stock, 200 mg/mL) 45 *µ* L of H₂O was added for 10-fold dilution, and then 19.6 *µ*L of the diluted solution (20 mg/mL) was taken for mixing with 32.4 *µ*L of DMSO and 78 *µ*L of ACN (with 0.5% HF). After 10 minutes of shaking at 4°C, additional bare silica nanoparticles were added into the solution for depleting the HF residue. Next, the solution was centrifuged for 10 minutes at 14,000 rpm; 120 µL of supernatant was taken for HPLC analysis.

### In vivo imaging system (IVIS) for detecting EPR effect and biodistribution of nanoparticles

*In vivo* biodistribution images of nanoparticles were obtained from the IVIS imaging system (Lumina). The Balb/c mice (4-week old) were purchased from BioLASCO. The tumorbearing mice were established by subcutaneous injection with 4T1 (ATCC ^{®}CRL-2539TM) tumor cells for heterotopic implantation. After the 4T1 cells grew for 2-3 weeks, the sample in PBS was intravenously injected. After 24 hours from injection, the major organs (heart, lung, spleen, liver and kidney) as well as tumors, urine and blood were carefully collected, and the fluorescence image and intensity of the collected samples were acquired by the IVIS Imaging System.

### Two-photon fluorescence microscopy for detecting nanoparticles in brain vessel.

Healthy ICRmice (27-30 g) were intravenously injected with 200 mg/kg nanoparticles, and dynamic imaging of the earlobes of mice was conducted by multi-photon microscopy (Olympus FVMPE-RS) with tunable excitation wavelengths (800-1000 nm). After nanoparticles were no longer circulated in the cerebral blood vessels, the mice were anaesthetized and a skull removal (craniotomy) procedure was then conducted. In this study, normal saline was used instead of placing a glass cover on the surface of the brain for shortterm observation. For imaging the blood vasculature, 0.6 mL of 2.5% (w/v) fluorescein isothiocyanate dextran (FITC-dextran, Mw: 70 kDa) was dissolved in sterile saline which was I.V injected into the mice. The depth profile imaging of nanoparticles in the mouse cerebrum was collected from 0 to 300 µm below the surface of the brain (axial spacing is 1 µm).

### Determination of the drug combination dose ratio by in-vitro anti-tumor synergistic effect test

The synergistic cytotoxicity effect of the JM17 (curcumin analog) and Doxorubicin (Dox) was evaluated by alamar blue assay (Invitrogen). The 5×10⁴ of MCF7/ADR cells (Dox resistant breast cancer cells) per well were seeded in 24-well plates for proliferation assays. The JM17 in various concentrations, Dox in various concentrations, and JM17 + Dox with different combination dose ratio were incubated with cells. After incubation for 48 hours, the cells were washed twice with a culture medium followed by incubation with the alamar blue reagent for 2 hours at 37°C. The fluorescence signals from the alamar blue assay were proportional to the number of live cells, and the signals (Ex/Em = 560/590) were measured using a microplate reader (Bio-Rad, model 68). Zheng-Jun Jin's methods (Q method) or the combination index (CI) theorem of Chou-Talalay was used to analyze the synergistic effect of drug combination.

### MCF7/ADR cancer animal model

Female BALB/c nude mice (5-6 weeks old) were purchased from the National Laboratory Animal Center. The mice were treated with sustained oral administration of estrogen through drinking water during the experiment period. The 5 × 10⁶ of MCF-7/ADR cells in 25 µL of PBS and 25 µL of Matrigel Matrix were subcutaneously xenografted in the left flanks of BALB/c nude mice. After the tumor growth continued for about one month (size ~ 50 mm³), all of the groups were injected on Day 0, 4 and 8 intravenously. The body weights and tumor volumes of the mice were measured twice a week.

### Synthetic Example 1

### Pore expanded MSN-PEG+TA (exMSN-PEG-TA) synthesis

Pore-expanded mesoporous silica nanoparticles (exMSNs) possess well-defined structures, large pores and high density of surface silanol groups which can be modified with a wide range of organic functional groups. Initially, 0.386 g of CTAB was dissolved in 160g of ammonium hydroxide solution (0.22M) at the desired temperature (50°C) in a sealed beaker. After 10 minutes, the 16.2mL diluted decane alcohol solution (7.4% v/v) was added and the mixture was stirred continuously for at least 8 hours (adding decane as oil phase for expanding the pore size). Afterwards, the sealed lid was removed, and the 660µL of TEOS in 2.64 mL ethanol was introduced into the mixture for an additional 1 hour of stirring. Next, the 550µL (2-[methoxy(polyethyleneoxy)propyl]- trimethoxysilane) (PEG) and 300µL N-[3-(trimethoxysilyl)propyl]-N,N,N- trimethylammonium chloride (TA) in 3mL ethanol were introduced into the reaction system. After the mixture was stirred for 30 minutes, the solution was aged at the desired temperature (50°C) without stirring overnight. Then the solution was filtrated through the 0.45µm and 0.22µm filter for removing the thin-film byproduct, after which the filtrate was sealed and placed in an oven at 80°C for 24 hours of hydrothermal treatment. The as-synthesized sample was washed and collected by cross-flow filtration. For removing the surfactant in the pores of the nanoparticles, the as-synthesized sample was incubated in 50 mL of acidic ethanol containing 848µL (first time) and 50µL (second time) of hydrochloric acid (37%) for one hour of extraction respectively at 60°C. The products were washed and harvested by cross-flow filtration and then stored in 90% ethanol.

### NH2+COOH-exMSN-PEG Synthesis

Initially, 0.386 g of CTAB was dissolved in 160g of ammonium hydroxide solution (0.22M) at the desired temperature (50°C) in a sealed beaker. After 10 minutes, the 16.2 mL diluted decane alcohol solution (7.4% v/v) was added and the mixture was stirred continuously for at least 8 hours. Afterwards, the sealed lid was removed, and the 700µL of TEOS, 50µL of 3-(2-aminoethyl amino)- propyltrimethoxysilane (AAS) and 5µL of N-(trimethoxysilyl-propyl) ethylenediamine triacetic acid trisodium salt (EDTAS) in 3.35 mL ethanol were introduced into the mixture for 1 hour of stirring. Next, the 550µL of PEG in 3mL ethanol was introduced into the reaction system. After the mixture was stirred for 30 minutes, the solution was aged at the desired temperature (50°C) without stirring overnight. Then the solution was filtrated through the 0.45µm and 0.22µm filter for removing the thin-film byproduct, and then the filtrate was sealed and placed in an oven at 80 °C for 24 hours of hydrothermal treatment. The as-synthesized sample was washed and collected by cross-flow filtration. For removing the surfactant in the pores of the nanoparticles, the as-synthesized sample was incubated in 50 mL of acidic ethanol containing 848µL (first time) and 50µL (second time) of hydrochloric acid (37%) for for one hour of extraction respectively at 60°C. The products were washed and harvested by cross-flow filtration and then stored in 90% ethanol.

### BisTESB-exMSN-PEG+TA Synthesis (with different TEOS/BisTESB ratio)

Initially, 0.386 g of CTAB was dissolved in a 160g of ammonium hydroxide solution (0.22M) at the desired temperature (50°C) in a sealed beaker. After 10 minutes, the 16.2mL diluted decane alcohol solution (7.4% v/v) was added and the mixture was stirred continuously for at least 8 hours. After that, the sealed lid was removed, and then the 583.3µL of TEOS/200µL of 1,4-bis(triethoxysilyl)benzene (BisTESB) (4:1), 636.4µL of TEOS/109µL of BisTESB (9:1) or 656.3µL of TEOS/75µL of BisTESB (14:1) in 2.8 mL ethanol were introduced into the mixture for 1 hour of stirring. Next, the 550µL PEG and 300µL TA in 3mL ethanol were introduced into the reaction system. After the mixture was stirred for 30 minutes, the solution was aged at the desired temperature (50°C) without stirring overnight. Then the solution was filtrated through the 0.45µm and 0.22µm filter for removing the thin-film byproduct, and then the filtrate was sealed and placed in an oven at 80°C for 24 hours of hydrothermal treatment. The as-synthesized sample was washed and collected by cross-flow filtration For removing the surfactant in the pores of the nanoparticles, the as-synthesized sample was incubated in 50 mL of acidic ethanol containing 848µL (first time) and 50µL (second time) of hydrochloric acid (37%) for one hour of extraction respectively at 60°C. The products were washed and harvested by cross-flow filtration and then stored in 90% ethanol.

### Phenyl-exMSN-PEG+TA synthesis (with different TEOS/Phenyl-silane ratio)

The exMSN structure is formed by the co-condensation of inorganic tetraethoxysilanes (TEOS) and organic trimethoxyphenylsilane (TMPS), which can render the hydrophobicity to the exMSN pores and improve the hydrophobic drug loading efficiency. The monodisperse Phenyl-exMSN-PEG+TA with the expanded pores was prepared by additionally introducing the decane to the ammonia solution with highly diluted surfactant. Initially, 0.386 g of CTAB was dissolved in 160g of ammonium hydroxide solution (0.22M or 0.35M) at the desired temperature (50°C) in a sealed beaker. After 10 minutes, the 16.2mL diluted decane alcohol solution (7.4% v/v) was added and the mixture would be stirred continuously for at least 8 hours. After that, the sealed lid was removed, and then added 2.5mL of diluted APTMS alcohol solution (10.7mM) and 350µL to 700µL of the TEOS with 37µL, 47µl, 56µL, or 112µL of the TMPS (the molar ratio of TEOS/TMPS = 16:1, 13:1, 11:1, and 5:1, the TEOS:TMPS ratio can be changed to modulate the hydrophobicity of the pore surface for different applications or loaded drugs) mixed in the 1.9mL to 3.3mL ofEtOH were added to the solution under vigorous stirring. After 3 to 60 minutes, 140µL to 350µL of ethanolic TEOS in the 0.56 mLto 1.4mL of EtOH was added for some the conditions (TEOS/TMPS = 16:1, 13:1 and 11:1); After 1 to 2 hours of the reaction, the 550µL to 1000µL of PEG and 155.8µl to 450µL TA mixed in 2mL to 3mL of EtOH were introduced into the reaction. After the mixture was stirred for 60 minutes, the mixture was aged at the desired temperature (50°C) without stirring overnight. Then the solution was filtrated through the 0.45µm and 0.22µm filter for removing the thin-film byproduct, and then the filtrate was sealed and placed in an oven at 80°C for 24 hours of hydrothermal treatment. The as-synthesized sample was washed and collected by cross-flow filtration. For removing the surfactant in the pores of the Phenyl-exMSN-PEG+TA, the as-synthesized sample was incubated in 50 mL of acidic ethanol containing 848µL (first time) and 50µL (second time) of hydrochloric acid (37%) for one hour of extraction respectively at 60°C. The products were washed and harvested by cross-flow filtration and then stored in 90% ethanol. The particle size of the Phenyl-exMSN-PEG+TA with various internal surface modifications measured via Dynamic Light Scattering (DLS) in different solution environments is shown in Table 1. DLS results show that all MSNs dispersed well within the range from about 40 nm to 70 nm in H₂O but the dispersity of high phenyl-introduced particle (TEOS:TMPS = 5:1) was influenced in PBS buffer. It means that partial phenyl group may have been modified on the surface of high phenyl-introduced particle lead to influence the dispersity of particle.

**Table 1.**

| TEOS : TMPS Ratio (mole) | DLS (size, nm) | |
|---|---|---|
| | in H₂O | in PBS |
| 16 : 1 | 46.0 | 53.7 |
| 13 : 1 | 47.2 | 47.8 |
| 11 : 1 (NTT2_151) | 56.7 | 62.5 |
| 11 : 1 (NTT2_131) | 40.7 | 41.5 |
| 5 : 1 | 68.1 | 3451 |

### Synthetic Example 2

### Phenyl-MSN-PEG+TA Synthesis (with different TEOS/Phenyl-silane ratio )

The Phenyl-MSN-PEG+TA was prepared using an ammonia base-catalyzed method under highly dilute and low surfactant conditions. The amount of phenyl group in the Phenyl-MSN-PEG+TA was adjusted by introducing the different TEOS and TMPS amounts into the reaction (the molar ratio of TEOS/TMPS = 29:1, 26:1, 21:1, 20:1, 15:1 and 11:1). Typically, 0.29 g of CTAB was dissolved in 150 mL of ammonium hydroxide solution (0.171M or 0.205M) at the desired temperature (50°C) in a sealed beaker. After 15-minutes of stirring, the sealed membrane was removed, and then 250µL of ethanolic TEOS with 16µL, 20µL, 24µL, 32µL or 40µL TMPS mixed in the 1mL EtOH were added to the solution under vigorous stirring. After 10 to 30 minutes, 250µL or 300µL of ethanolic TEOS in the 1mL or 1.2mL of EtOH was added. After 1 to 2.5 hours of the reaction, some conditions of them were additionally added with the 50µL of ethanolic TEOS dissolved in the 200µL of EtOH for 10 minutes, and then the 550µL to 825µL of PEG and 300µL to 450µL TA mixed in 2mL to 2.6mL of EtOH were introduced into the reaction. After the mixture was stirred for 1 hour, the mixture was aged at desired temperature (50°C) without stirring for at least 12 hours. And then the solution was sealed and placed in an oven at 70°C for 24 hours of hydrothermal treatment. The as-synthesized sample was washed and collected by Cross-Flow filtration. For removing the surfactant in the pores of the Phenyl-MSN-PEG+TA, the as-synthesized sample was collected in 40 mL of acidic ethanol containing 678µL (first time) and 40µL (second time) of hydrochloric acid (37%) for 1 hour extraction respectively at 60°C. The products were washed and harvested by Cross-Flow filtration and then stored in 90% ethanol. The particle size of the Phenyl-MSN-PEG+TA with various internal surface modifications measured via Dynamic Light Scattering (DLS) in different solution environments is shown in Table 2. DLS results show that all MSNs dispersed well within the range from about 30 nm to 50 nm in H₂O but the dispersity of high phenyl-introduced particle (TEOS:TMPS = 11:1 and 15: 1) was influenced in PBS buffer. It means that partial phenyl group may have been modified on the surface of high phenyl-introduced particle lead to influence the dispersity of particle.

**Table 2.**

| TEOS : TMPS Ratio (mole) | DLS (size, nm) | |
|---|---|---|
| | in H₂O | in PBS |
| 29: 1 | 37.0 | 40.1 |
| 26 : 1 | 40.7 | 44.9 |
| 21 : 1 | 39.0 | 41.3 |
| 20 : 1 | 36.5 | 43.1 |
| 15 : 1 | 48.9 | 785.1 |
| 11 : 1 | 32.9 | 2570 |

### Synthetic example 3

### C8-MSN-PEG+TA synthesis (with different TEOS/C8-silane ratio)

The C8-MSN-PEG+TA was prepared by using an ammonia base-catalyzed method with a highly dilute and low surfactant level solution. The octyl group content in the C8-MSN-PEG+TA was adjusted by the total TEOS and C8-silane amounts for the reaction (the molar ratio of TEOS/C8-silane varies from 20:1, 15:1, 10:1 and 5:1). Typically, 0.29 g of CTAB was dissolved in 150 mL of ammonium hydroxide solution (0.205M) at the desired temperature (50°C) in a sealed beaker. After 15-minutes of stirring, the sealed membrane was removed, and then 250µL of ethanolic TEOS with 39.2 µL, 52.2 µL, 78.4 µL or 156.8 µL C8-silane mixed in the 1 mL of EtOH were added to the solution under vigorous stirring. Another addition of 300 µL of ethanolic TEOS in the 1.2 mL of EtOH was introduced 1 hour later. After 3 hours of the reaction, 825 µL of PEG and 450 µL TA mixed in 2.6 mL of EtOH were introduced for further reaction. After the mixture was stirred for 1 hour, the mixture was aged at desired temperature (50°C) without stirring for at least 12 hours. Then, the resulted solution was sealed and placed in an oven at 70°C for 24 hours of hydrothermal treatment. Finally, the as-synthesized sample was washed and the products were collected by Cross-Flow system. To remove the surfactant in the pores of the C8-MSN-PEG+TA, the as-synthesized products were introduced into 40 mL of acidic ethanol containing 678 µL (first time) and 40 µL (second time) of hydrochloric acid (37%) for two times of 1 hour of extraction at 60°C. The products were washed and harvested by Cross-Flow system to give the final products and the final products were finally stored in 90% ethanol. The particle size of C8-MSN-PEG+TA with various internal surface modifications measured via Dynamic Light Scattering (DLS) in different environments is listed in Table 3. All MSNs dispersed well, having a size within the range from about 25 nm to 40 nm in H₂O; however, the dispersity of high C8-introduced particles (TEOS:C8-silane = 10:1 and 5:1) in PBS buffer was influenced. The results show that parts of the alkyl groups may have been attached on the outer surface of C8-introduced particle when a higher amount of C8-silane is used, thereby decreasing the dispersity of particles.

**Table 3.**

| TEOS : C8-silane Ratio (mole) | DLS (size, nm) | |
|---|---|---|
| | in H₂O | in PBS |
| 20: 1 | 31.5 | 40.0 |
| 15 : 1 | 29.8 | 76.2 |
| 10 : 1 | 39.3 | 972.7 |
| 5 : 1 | 32.8 | 2697 |

### Example 4

### Doxorubicin and JM17 Co-Loading in pore-modified MSN and exMSNs

The 50mg of exMSNs was first soaked in the NaHCOs solution (pH 9.95) for 30 minutes, and then the solution was concentrated by Vivaspin^{®} Turbo 15 to give a concentrate. Afterwards, the concentrate was diluted with DI water and then the diluted solution was concentrated again. To the thus concentrated solution doxorubicin (Dox) solution was added and the mixture was shaken for 30 minutes at 4°C. After that, the mixture solution (containing Dox and exMSNs) was slowly dropped into JM17 solution (in 100% DMSO) with continuous shake for 2 hours at 4°C. Next, to the mixture DI water was added along with vigorous shaking for decreasing the DMSO concentration to the level of maximum DMSO tolerance of the Vivaspin membrane. Before the concentrating and washing process, the solution is centrifuged at a low rate (3500g) for 10 minutes for separating partial JM17 precipitate (if needed). Finally, the product is stored in the DI water. The JM17 and Dox loaded amounts in exMSNs can be adjusted by using JM17 and Dox solution at different concentrations during the drug loading process.

All kinds of exMSNs were loaded with JM17 and Dox by the method mentioned above. It was noted that JM17 and Dox cannot be loaded simultaneously into the exMSN-PEG+TA and NH2+COOH-exMSN-PEG, which means that the methods of only expanding the pore size of MSN or modifying the pore surface - with positively charged, negatively charged, H-bond donors or H-bond acceptors - cannot make the MSN adsorb the hydrophobic drug (JM17) and hydrophilic drug (Dox) simultaneously. Even though the BisTESB-exMSN-PEG+TA nanoparticles were constructed to bear hydrophobic phenyl group in the structure, they still cannot encapsulate both drugs into the particles. In contrast, phenyl-exMSN-PEG+TA has the ability to encapsulate JM17 and Dox simultaneously, which means that phenyl group was modified on the surface of the pores thereby enhancing the drug loading ability. The hydrophobic phenyl group, derived from the TMPS, in the phenyl-exMSN-PEG+TA plays a critical role in improving hydrophobic drug loading efficiency; hence, the TMPS/TEOS ratio of the phenyl-exMSN-PEG+TA synthesis would be optimized in order to achieve higher drug loading capability without influencing the material dispersity in the solution. Therefore, the TEOS/TMPS ratios of 5:1, 11:1, 13:1 and 16:1 are selected for the exMSN synthesis to evaluate the effect. The results demonstrate that the nanoparticles synthesized by TEOS/TMPS ratio of 11:1 exhibit the best drug loading ability (DOX: 1-5%; JM17: 1-5%) and still maintain excellent dispersity in PBS (DLS size/PDI: 44.4 nm/0.095). On the other hand, nanoparticles might severely aggregate during the drug loading process if they are synthesized with a lower TEOS/TMPS ratio, e.g., 5:1, and the aggregation may be caused by phenyl groups attached on the surface of particles, which allow hydrophobic drugs to be also attached onto the surface of particle, rather than within the space of the pore. The aggregated nanoparticles (DLS size >200 nm even in H₂O) would cause low drug loading efficiency, making them unable to be utilized for in-vivo experiments due to poor blood circulation and a risk of vascular obstruction. Further, nanoparticles with a low TEOS/TMPS ratio (16:1) exhibit good suspension in solution, but they adsorb JM17 less efficiently; the loading capacity is too low to be used.

Phenyl-MSNs-PEG+TA can also encapsulate drugs in higher drug loading capability without influencing the material dispersity. Six TEOS/TMPS ratios, 29:1, 26:1, 21:1, 20:1, 15:1 and 11:1, are selected for the pore-modified MSN synthesis. The nanoparticles synthesized with TEOS/TMPS ratio ranging from 29:1 to 20:1 exhibit high drug loading ability (>3% loading weight percent) and still can maintain excellent dispersity in the PBS (DLS size <60 nm). On the other hand, nanoparticles would severely aggregate after the loading process if they are synthesized with the TEOS/TMPS ratio of 15:1 or 11:1, and the DLS size of aggregated nanoparticles is > about 200 nm in H₂O.

Phenyl-exMSN-PEG+TA, Phenyl-MSN-PEG+TA, and C8-MSN-PEG+TA also can encapsulate different hydrophobic drugs such as ixabepilone, paclitaxel, irinotecan. According to the testing results, through the specific surface modification of pores and modulation of the ratio of functional groups, MSN and exMSN can have the ability of encapsulating multiple drugs with different physico-chemical properties in the same particle.

### Example 5

### Phenyl-group concentration of phenyl-exMSN

In the phenyl-MSN-PEG+TA and phenyl-exMSN-PEG+TA synthesis process, TEOS and TMPS at different molar ratios were used for the reaction for modulating the surface modification of pores. For quantifying the phenyl functional group on the internal surface of MSN and exMSN nanoparticles, the elemental composition of phenyl-MSN particles was measured by elemental analyzer. The number of phenyl group per mg particle is derived from the mass percent of carbon of phenyl-MSN and phenyl-exMSN particles. The number of phenyl group of phenyl (29:1), (21:1) and (11:1)-MSN derived from elemental analysis is about 6.68 × 10¹⁷, 7.9 × 10¹⁷ ,1.03 × 10¹⁸ (molecule/mg) respectively and the number of phenyl group of phenyl(16:1), (11:1) and (5:1)exMSN derived from elemental analysis is about 8.08 × 10¹⁷, 1.04 × 10¹⁸ ,1.62 × 10¹⁸ (molecule/mg) respectively. The particle concentration (number/mg) of sample solution was measured by nanoparticle tracking analysis. The concentration of phenyl(29:1), (21:1), (11:1)-MSN is 2.29 × 10¹², 2.27 × 10¹² ,3.02 × 10¹² (particle/mg) respectively and the concentration of phenyl(16:1), (11:1), (5:1)-exMSN is 2.98 × 10¹², 3.97 × 10¹² ,4.44 × 10¹² (particle/mg) respectively. According to the results, the amount of phenyl group on each nanoparticle of phenyl(29:1), (21:1), (11:1)-MSN is 2.92 × 10⁵, 2.85 × 10⁵ ,3.41 × 10⁵ (number of phenyl/particle) respectively and the amount of phenyl group on each nanoparticle of phenyl(16:1), (11:1), (5:1)-exMSNis 2.71 × 10⁵, 2.61 × 10⁵ ,3.64 × 10⁵ (number of phenyl/particle) respectively.

### Example 6

### In-vitro synergistic effect of JM17 and Dox codelivery by exMSN

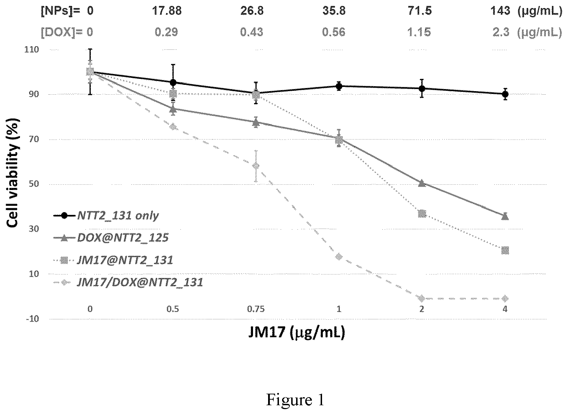

According to the method of determination of the drug combination dose ratio, a specific ratio range of JM17:Dox (1:0.4 - 1 :0.6) showed higher synergistic effect on inhibition of Dox resistant cancer cells (MCF-7/ADR). Therefore, a specific JM17: Dox dose ratio encapsulated by exMSN was synthesized, and the synergistic cytotoxicity effect of the particle was evaluated. The 5×10⁴ of MCF7/ADR cells per well were seeded in 24-well plates for proliferation assays. The four groups including (1) Phenyl-exMSN-PEG+TA (NTT2_131), (2) DOX@Phenyl-exMSN-PEG+TA (Dox@NTT2_131), (3) JM17@Phenyl-exMSN-PEG+TA (JM17@NTT2_131) and (4) JM17+DOX@Phenyl-exMSN-PEG+TA (JM17/Dox@NTT2_131) in various concentrations were incubated with cells. The JM17/Dox@NTT2_131 treated group showed efficient inhibition of the MCF-7/ADR cancer cells; it was revealed that JM17 and Dox co-delivery by nanoparticle can exhibit a good synergistic effect in vitro, the results are shown in figure 1.

### Example 7

### In-vivo anti MCF7/ADR tumor efficacy

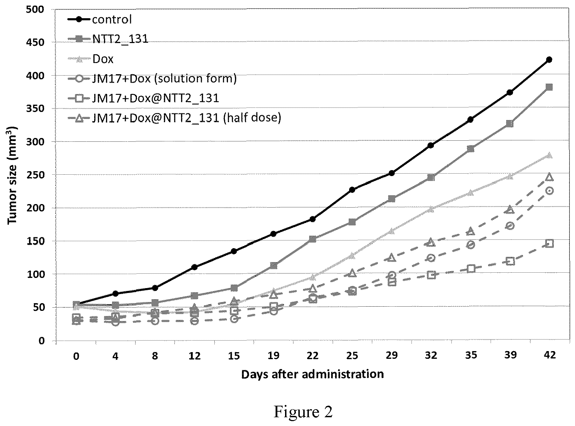

Female BALB/c nude mice were randomly allocated into six groups (n = 5~6): (1) Control, (2) DOX, (3) JM17+Dox (solution form), (4) JM17+Dox@NTT2_131, (5) JM17+Dox@NTT2_131 (half dose), and (6) NTT2_131 (particle only). The groups (1)-(4) had the same Dox and JM17 dose. The 5 × 10⁶ of MCF-7/ADR cells (Dox resistant breast cancer cells) in 25 µL of PBS and 25 µL of Matrigel Matrix were subcutaneously xenografted in the left flanks of BALB/c nude mice. After the tumor growth continued for about one month (size ~ 50 mm3), all of the groups were injected on day 0, 4 and 8 by intravenous injection, and the half dosage group were injected with an additional three doses on Days 12, 16 and 20. Body weights and tumor volumes of the mice were measured twice a week. According to the results, JM17 and Dox co-delivery showed synergistic effect in anti-tumor efficacy. The JM17/Dox@NTT2_131 (half dose) group had comparable anti-tumor efficacy and less toxicity than the JM17 + Dox (solution form) group; furthermore the JM17/Dox@NTT2_131 group (regular dose) had the highest anti-tumor efficacy (Figure 2). This result demonstrated that the nanoparticle can provide several advantages for multidrug co-delivery compared with combinations of free drugs (solution form) such as: (1) nanoparticles can deliver hydrophobic and hydrophilic drugs at the same time and maintain the optimized synergistic drug ratio in a single nanoparticle, (2) nanoparticles can elongate circulation time and enhance tumor targeting ability of drugs, and (3) drug encapsulated nanoparticles can deliver multidrugs into a target cell simultaneously that will normalize the pharmacokinetic difference between drugs. These advantages of nanoparticle formulation will enhance the synergistic effect of combination drugs on anti-tumor efficacy.

### Example 8

### The blood-brain barrier penetration capability of phenyl-exMSN-PEG+TA particle

Delivery of therapeutic drugs into the brain is still a major challenge because of the blood-brain barrier (BBB). Nanoparticles smaller than 100 nm provide advantages in improving drug transport across the BBB. The 30 nm phenyl-exMSN modified with positively charged TA molecules on the surface, may have the potential of crossing through the BBB. To understand BBB penetration capability of the nanoparticles in vivo, we used two-photon fluorescence spectroscopy to monitor the distribution of nanoparticles in vessels of the brains in mice. The fluorescent-labelled nanoparticles were administered through the tail vein. Two days after injection, we detected brain vessels located within a depth of 0-300 µm from the surface. Meanwhile, FITC-dextran was injected through IV for mapping the angioarchitecture and revealing the boundary of blood vessel walls. If the fluorescence signal from nanoparticles overlapped with the FITC-dextran's signal (green signal), it appeared as a yellow signal (in some cases it will be red), meaning that the nanoparticle was not in the blood vessel, and may have crossed the BBB into the brain tissue. The 3D images of the brain vessel showed numerous red signals from phenyl-exMSN-PEG+TA nanoparticles distributed in the blood vessel wall and the brain tissue area. The results indicated that 30 nm phenyl-exMSN-PEG+TA exhibits a potential to cross the BBB into the brain tissue.

A person of ordinary skill in the art of the subject invention should understand that variations and modification may be made to the teaching and the disclosure of the subject invention without departing from the spirit and scope of the subject application. Based on the contents above, the subject application intends to cover any variations and modification thereof with the proviso that the variations or modifications fall within the scope as defined in the appended claims or their equivalents.

## Claims

1. A mesoporous silica nanoparticle, **characterized in that** it comprises organic modification on the surface of its pores and has a hydrodynamic size in water of no greater than 100 nm and a hydrodynamic size in a medium, of no greater than 150 nm, measured by Dynamic Light Scattering (DLS) with a Malvern Zetasizer Nano ZS, wherein the organo modification comprises at least one terminal hydrocarbyl moiety, and wherein the medium is biologically similar to or equivalent to phosphate buffered saline (PBS).

2. The mesoporous silica nanoparticle of claim 1, wherein the pore size of the mesoporous silica nanoparticle is no greater than 50 nm.

3. The mesoporous silica nanoparticle of claim 1, wherein the hydrodynamic size in the medium of the mesoporous silica nanoparticle is no greater than 100 nm.

4. The mesoporous silica nanoparticle of claim 1, wherein the terminal hydrocarbyl moiety comprises a terminal aromatic moiety, a terminal aliphatic moiety or combinations thereof.

5. The mesoporous silica nanoparticle of claim 4, wherein the terminal aromatic moiety is derived from a silica source selected from the group consisting of trimethoxyphenylsilane (TMPS), triethoxyphenylsilane, diphenyldiethoxysilane, 1-naphthyl trimethoxysilane, 2-hydroxy-4-(3-triethoxy silylpropoxy)diphenylketone, O-4-methylcoumarinyl-N-[3-(triethoxysilyl)propyl]carbamate, 7-triethoxysilylpropoxy-5-hydroxyflavone, 3-carbazolylpropyltriethoxysilane, bis(2-diphenylphosphinoethyl)methylsilylethyltriethoxysilane and 2-(diphenylphosphino)ethyl triethoxysilane.

6. The mesoporous silica nanoparticle of claim 4, wherein the terminal aliphatic moiety is derived from a silica source selected from the group consisting of propyltriethoxysilane n-butyltriethoxysilane, pentyltriethoxysilane, hexyltriethoxysilane, heptyltriethoxysilane, octyltriethoxysilane, nonyltriethoxysilane, decyltriethoxysilane, undecyltriethoxysilane, dodecyltriethoxysilane, cyclopropyltriethoxysilane, cyclobutyltriethoxysilane, cyclopentyltriethoxysilane, cyclohexyltriethoxysilane, cycloheptyltriethoxysilane, cyclooctyltriethoxysilane, propyltrimethoxysilane n-butyltrimethoxysilane, pentyltrimethoxysilane, hexyltrimethoxysilane, heptyltrimethoxysilane, octyltrimethoxysilane, nonyltrimethoxysilane, decyltrimethoxysilane, undecyltrimethoxysilane, dodecyltrimethoxysilane, cyclopropyltrimethoxysilane, cyclobutyltrimethoxysilane, cyclopentyltrimethoxysilane, cyclohexyltrimethoxysilane, cycloheptyltrimethoxysilane and cyclooctyltrimethoxysilane.

7. The mesoporous silica nanoparticle of claim 1, wherein the amount of terminal hydrocarbyl moiety per particle is less than 1 × 10⁶ molecule/particle.

8. The mesoporous silica nanoparticle of claim 1, wherein it further comprises at least one hydrophobic bioactive ingredient or at least one hydrophilic bioactive ingredient loaded within the pores.

9. The mesoporous silica nanoparticle of claim 8, wherein the hydrophobic bioactive ingredient is a small molecule, a chemo-drug, an enzyme, a protein drug, an antibody, a vaccine, an antibiotic, a nucleotide drug or combinations thereof.

10. The mesoporous silica nanoparticle of claim 8, wherein it further comprises at least one hydrophilic or hydrophobic bioactive ingredient loaded within the pores.

11. The mesoporous silica nanoparticle of claim 10, wherein the bioactive ingredients have synergistic effect.

12. The mesoporous silica nanoparticle of any of the claims 1-11 for use in a method for delivering bioactive ingredient(s) to a subject, comprising:
(1) loading the bioactive ingredients within the pores of the mesoporous silica nanoparticle of any of claims 1-11; and
(2) administering the mesoporous silica nanoparticle described in (1) to the subject.

13. The mesoporous silica nanoparticle for use in a method according to claim 12, wherein the mesoporous silica nanoparticle is delivered to penetrate blood brain barrier.

14. The mesoporous silica nanoparticle for use in a method according to claim 12, wherein the mesoporous silica nanoparticle is delivered to penetrate blood ocular barrier.

15. A method for preparing a mesoporous silica nanoparticle (MSN) with internal surface organic modification on the pores, comprising the steps of:
(a) providing an alkaline solution containing a surfactant to form micelles;
(b) adding a first silica source and a second silica source which provides a terminal hydrocarbyl moiety into the solution, wherein the molar ratio of the first silica source and the second silica source is no less than 5: 1;
(c) conducting hydrothermal treatment to the solution; and
(d) extracting and optionally purifying the MSNs from the solution.

16. The method of Claim 15, wherein the method further comprises at least one of the following steps:
(e) introducing oil phase into the solution after step (a) and before step (b) for pore extension;
(f) adding a further silica source after step (b); and
(g) conducting surface modification of the external surface of the MSNs, wherein the surface modification is conducted after step (b), or after step (f) if step (f) is conducted.

17. The method according to Claim 15, wherein the surfactant is a cationic surfactant, an anionic surfactant, a non-ionic surfactant or any combinations thereof.

18. The method according to Claim 15, wherein the first silica source comprises tetraethoxysilane (TEOS), tetramethoxysilane (TMOS), sodium silicate or a mixture thereof.

19. The method according to Claim 15, wherein the second silica source is selected from the group consisting of trimethoxyphenylsilane (TMPS), triethoxyphenylsilane (TEPS), diphenyldiethoxysilane, 1-naphthyl trimethoxysilane, 2-hydroxy-4-(3-triethoxy silylpropoxy)diphenylketone, O-4-methylcoumarinyl-N-[3- (triethoxysilyl)propyl]carbamate, 7-triethoxysilylpropoxy-5-hydroxyflavone, 3-carbazolylpropyltriethoxysilane, bis(2-diphenylphosphinoethyl)methylsilylethyltriethoxysilane, 2-(diphenylphosphino)ethyl triethoxysilane, propyltriethoxysilane n-butyltriethoxysilane, pentyltriethoxysilane, hexyltriethoxysilane, heptyltriethoxysilane, octyltriethoxysilane, nonyltriethoxysilane, decyltriethoxysilane, undecyltriethoxysilane, dodecyltriethoxysilane, cyclopropyltriethoxysilane, cyclobutyltriethoxysilane, cyclopentyltriethoxysilane, cyclohexyltriethoxysilane, cycloheptyltriethoxysilane, cyclooctyltriethoxysilane, propyltrimethoxysilane n-butyltrimethoxysilane, pentyltrimethoxysilane, hexyltrimethoxysilane, heptyltrimethoxysilane, octyltrimethoxysilane, nonyltrimethoxysilane, decyltrimethoxysilane, undecyltrimethoxysilane, dodecyltrimethoxysilane, cyclopropyltrimethoxysilane, cyclobutyltrimethoxysilane, cyclopentyltrimethoxysilane, cyclohexyltrimethoxysilane, cycloheptyltrimethoxysilane and cyclooctyltrimethoxysilane.

20. The method according to Claim 15, wherein the oil phase described in step (e) comprises substituted or unsubstituted (cyclo)alkane(s), substituted or unsubstituted aromatic solvent(s) or combinations thereof.

21. A mesoporous silica nanoparticle, which is prepared by the method of any of Claims 15 to 20.

## Patentansprüche

1. Mesoporöses Siliciumdioxidnanopartikel, **dadurch gekennzeichnet, dass** es eine organische Modifikation auf der Oberfläche seiner Poren umfasst und eine hydrodynamische Größe in Wasser von nicht mehr als 100 nm und eine hydrodynamische Größe in einem Medium von nicht mehr als 150 nm aufweist, gemessen durch dynamische Lichtstreuung (DLS) mit einem Malvern Zetasizer Nano ZS, wobei die Organomodifikation mindestens einen endständigen Hydrocarbylrest umfasst, und wobei das Medium biologisch ähnlich oder äquivalent zu phosphatgepufferter Kochsalzlösung (PBS) ist.

2. Mesoporöses Siliciumdioxidnanopartikel nach Anspruch 1, wobei die Porengröße des mesoporösen Siliciumdioxidnanopartikels nicht größer als 50 nm ist.

3. Mesoporöses Siliciumdioxidnanopartikel nach Anspruch 1, wobei die hydrodynamische Größe in dem Medium des mesoporösen Siliciumdioxidnanopartikels nicht größer als 100 nm ist.

4. Mesoporöses Siliciumdioxidnanopartikel nach Anspruch 1, wobei der endständige Hydrocarbylrest ein endständiger aromatischer Rest, ein endständiger aliphatischer Rest oder Kombinationen davon umfasst.

5. Mesoporöses Siliciumdioxidnanopartikel nach Anspruch 4, wobei der endständige aromatische Rest von einer Siliciumdioxidquelle abgeleitet ist, die aus der Gruppe ausgewählt ist, bestehend aus Trimethoxyphenylsilan (TMPS), Triethoxyphenylsilan, Diphenyldiethoxysilan, 1-Naphthyltrimethoxysilan, 2-Hydroxy-4-(3-triethoxysilylpropoxy)diphenylketon, O-4-methylcumaryl-N-[3-(triethoxysilyl)propyl]carbamat, 7-Triethoxysilylpropyl-5-hydroxyflavon, 3-Carbazolyltriethoxysilan, bis(2-Diphenylphosphinoethyl)methylsilyltrityltriethoxysilan und 2-(Diphenylphosphino)ethyltriethoxysilan.

6. Mesoporöses Siliciumdioxidnanopartikel nach Anspruch 4, wobei der endständige aliphatische Rest von einer Siliciumdioxidquelle abgeleitet ist, die aus der Gruppe ausgewählt sind, bestehend aus Propyltriethoxysilan-n-butyltriethoxysilan, Pentyltriethoxysilan, Hexyltriethoxysilan, Heptyltriethoxysilan, Octyltriethoxysilan, Nonyltriethoxysilan, Decyltriethoxysilan, Undecyltriethoxysilan, Dodecyltriethoxysilan, Cyclopropyltriethoxysilan, Cyclobutyltriethoxysilan, Cyclopentyltriethoxysilan, Cyclohexyltriethoxysilan, Cycloheptyltriethoxysilan, Cyclooctyltriethoxysilan, Propyltrimethoxysilan-n-butyltrimethoxysilan, Pentyltrimethoxysilan, Hexyltrimethoxysilan, Heptyltrimethoxysilan, Octyltrimethoxysilan, Nonyltrimethoxysilan, Decyltrimethoxysilan, Undecyltrimethoxysilan, Dodecyltrimethoxysilan, Cyclopropyltrimethoxysilan, Cyclobutyltrimethoxysilan, Cyclopentyltrimethoxysilan, Cyclohexyltrimethoxysilan, Cycloheptyltrimethoxysilan und Cyclooctyltrimethoxysilan.

7. Mesoporöses Siliciumdioxidnanopartikel nach Anspruch 1, wobei die Menge des endständigen Hydrocarbylrests pro Partikel weniger als 1 × 10⁶ Molekül/Partikel beträgt.

8. Mesoporöses Siliciumdioxidnanopartikel nach Anspruch 1, wobei es ferner mindestens einen hydrophoben bioaktiven Bestandteil oder mindestens einen hydrophilen bioaktiven Bestandteil umfasst, der innerhalb der Poren geladen ist.

9. Mesoporöses Siliciumdioxidnanopartikel nach Anspruch 8, wobei der hydrophobe bioaktive Bestandteil ein kleines Molekül, ein Chemo-Arzneimittel, ein Enzym, ein Proteinarzneimittel, ein Antikörper, ein Impfstoff, ein Antibiotikum, ein Nucleotidarzneimittel oder Kombinationen davon ist.

10. Mesoporöses Siliciumdioxidnanopartikel nach Anspruch 8, wobei es ferner mindestens einen hydrophilen oder hydrophoben bioaktiven Bestandteil umfasst, der innerhalb der Poren geladen ist.

11. Mesoporöses Siliciumdioxidnanopartikel nach Anspruch 10, wobei die bioaktiven Bestandteile eine synergistische Wirkung aufweisen.

12. Mesoporöses Siliciumdioxidnanopartikel nach einem der Ansprüche 1 bis 11 zur Verwendung in einem Verfahren zum Zuführen von bioaktivem/n Bestandteil(en) an ein Subjekt, umfassend:
(1) Laden der bioaktiven Bestandteile innerhalb der Poren des mesoporösen Siliciumdioxidnanopartikels nach einem der Ansprüche 1 bis 11; und
(2) Verabreichen des in (1) beschriebenen mesoporösen Siliciumdioxidnanopartikels an das Subjekt.

13. Mesoporöses Siliciumdioxidnanopartikel zur Verwendung in einem Verfahren nach Anspruch 12, wobei das mesoporöse Siliciumdioxidnanopartikel zugeführt wird, um die Blut-Hirn-Schranke zu durchdringen.

14. Mesoporöses Siliciumdioxidnanopartikel zur Verwendung in einem Verfahren nach Anspruch 12, wobei das mesoporöse Siliciumdioxidnanopartikel zugeführt wird, um die Blut-Okular-Barriere zu durchdringen.

15. Verfahren zum Herstellen eines mesoporösen Siliciumdioxidnanopartikels (MSN) mit einer internen organischen Oberflächenmodifikation auf den Poren, umfassend die Schritte:
(a) Bereitstellen einer alkalischen Lösung, die ein Tensid enthält, um Micellen auszubilden;
(b) Hinzufügen einer ersten Siliciumdioxidquelle und einer zweiten Siliciumdioxidquelle, die einen endständigen Hydrocarbylrest in die Lösung bereitstellt, wobei das Molverhältnis der ersten Siliciumdioxidquelle und der zweiten Siliciumdioxidquelle nicht weniger als 5 : 1 ist;
(c) Durchführen einer hydrothermalen Behandlung an die Lösung, und
(d) Extrahieren und optional Reinigen der MSNs aus der Lösung.

16. Verfahren nach Anspruch 15, wobei das Verfahren ferner mindestens einen der folgenden Schritte umfasst:
(e) Einführen von Ölphase in die Lösung nach Schritt (a) und vor Schritt (b) für eine Porenerweiterung;
(f) Hinzufügen einer weiteren Siliciumdioxidquelle nach Schritt (b); und
(g) Durchführen einer Oberflächenmodifikation der externen Oberfläche der MSNs, wobei die Oberflächenmodifikation nach Schritt (b) oder nach Schritt (f) durchgeführt wird, falls Schritt (f) durchgeführt wird.

17. Verfahren nach Anspruch 15, wobei das Tensid ein kationisches Tensid, ein anionisches Tensid, ein nichtionisches Tensid oder beliebige Kombinationen davon ist.

18. Verfahren nach Anspruch 15, wobei die erste Siliciumdioxidquelle Tetraethoxysilan (TEOS), Tetramethoxysilan (TMOS), Natriumsilicat oder eine Mischung davon umfasst.

19. Verfahren nach Anspruch 15, wobei die zweite Siliciumdioxidquelle aus der Gruppe ausgewählt ist, bestehend aus Trimethoxyphenylsilan (TMPS), Triethoxyphenylsilan (TEPS), Diphenyldiethoxysilan, 1-Naphthyl-trimethoxysilan, 2-Hydroxy-4-(3-triethoxysilylpropoxy)diphenylketon, O-4-methylcumarinyl-N-[3-(triethoxysilyl)propyl]carbamat, 7-Triethoxysilylpropoxy-5-hydroxyflavon, 3-Carbazolylpropyltriethoxysilan, bis(2-Diphenylphosphinoethyl)methylsilylethyltriethoxysilan, 2-(Diphenylphosphino)ethyltriethoxysilan, Propyltriethoxysilan-n-butyltriethoxysilan, Pentyltriethoxysilan, Hexyltriethoxysilan, Heptyltriethoxysilan, Octyltriethoxysilan, Nonyltriethoxysilae, Decyltriethoxysilan, Undecyltriethoxysilan, Dodecyltriethoxysilan, Cyclopropyltriethoxysilan, Cyclobutyltriethoxysilan, Cyclopentyltriethoxysilan, Cyclohexyltriethoxysilan, Cycloheptyltriethoxysilan, Cyclooctyltriethoxysilan, Propyltrimethoxysilan, n-Butyltrimethoxysilan, Pentyltrimethoxysilan. Hexyltrimethoxysilan, Heptyltrimethoxysilan, Octyltrimethoxysilan, Nonyltrimethoxysilan, Decyltrimethoxysilan, Undecyltrimethoxysilan, Dodecyltrimethoxysilan, Cyclopropyltrimethoxysilan, Cyclobutyltrimethoxysilan, Cyclopentyltrimethoxysilan, Cyclohexyltrimethoxysilan, Cycloheptyltrimethoxysilan und Cyclooctyltrimethoxysilan.

20. Verfahren nach Anspruch 15, wobei die in Schritt (e) beschriebene Ölphase substituierte(s) oder unsubstituierte(s) (cyclo)Alkan(e), substituierte(s) oder unsubstituierte(s) aromatische(s) Lösungsmittel oder Kombinationen davon umfasst.

21. Mesoporöses Siliciumdioxidnanopartikel, das durch das Verfahren nach einem der Ansprüche 15 bis 20 hergestellt wird.

## Revendications

1. Nanoparticule de silice mésoporeuse, **caractérisée en ce qu'**elle comprend une modification organique sur la surface de ses pores et présente une taille hydrodynamique dans l'eau non supérieure à 100 nm et une taille hydrodynamique dans un milieu, non supérieure à 150 nm, mesurée par diffusion dynamique de la lumière (DLS) avec un Malvern Zetasizer Nano ZS, dans lequel la-modification organique comprend au moins une fraction hydrocarbyle terminale, et dans lequel le milieu est biologiquement similaire ou équivalent à une solution saline tamponnée au phosphate (PBS).

2. Nanoparticule de silice mésoporeuse selon la revendication 1, dans laquelle la taille de pore de la nanoparticule de silice mésoporeuse n'est pas supérieure à 50 nm.

3. Nanoparticule de silice mésoporeuse selon la revendication 1, dans laquelle la taille hydrodynamique dans le milieu de la nanoparticule de silice mésoporeuse n'est pas supérieure à 100 nm.

4. Nanoparticule de silice mésoporeuse selon la revendication 1, dans laquelle la fraction hydrocarbyle terminale comprend une fraction aromatique terminale, une fraction aliphatique terminale ou des combinaisons de celles-ci.

5. Nanoparticule de silice mésoporeuse selon la revendication 4, dans laquelle la fraction aromatique terminale est dérivée d'une source de silice choisie dans le groupe constitué par le trimethoxyphenylsilane (TMPS), le triéthoxyphénylsilane, le diphényldiéthoxysilane, 1-naphtyltriméthoxysilane, 2-hydroxy-4-(3-triéthoxysilylpropoxy)diphénylcétone, O-4-méthylcoumarinyl-N-[3-(triéthoxysilyl) propyl] carbamate, 7-triéthoxysilylpropoxy-5-hydroxyflavone, 3-carb azolylpropyltriéthoxysilane, bis(2-diphénylphosphinoéthyl)méthylsilyléthyltriéthoxysilane and 2-(diphénylphosphino)éthyl triéthoxysilane.

6. Nanoparticule de silice mésoporeuse selon la revendication 4, dans laquelle la fraction aliphatique terminale est dérivée d'une source de silice choisie dans le groupe constitué par le propyltriéthoxysilane de n-butyltriéthoxysilane, le pentyltriéthoxysilane, l'hexyltriéthoxysilane, l'heptyltriéthoxysilane, l'octyltriéthoxysilane, le nonyltriéthoxysilane, le décyltriéthoxysilane, l'undécyltriéthoxysilane, le dodécyltriéthoxysilane, le cyclopropyltriéthoxysilane, le cyclobutyltriéthoxysilane, le cyclopentyltriéthoxysilane, le cyclohexyltriéthoxysilane, le cycloheptyltriéthoxysilane, le cycloheptyltriéthoxysilane, le cyclooctyltriéthoxysilane, le propyltriméthoxysilane n-butyltriméthoxysilane, le pentyltrimethoxysilane, l'hexyltriméthoxysilane, l'heptyltriméthoxysilane, l'octyltriméthoxysilane, le nonyltriméthoxysilane, le décyltriméthoxysilane, l'undécyltriméthoxysilane, le dodécyltriméthoxysilane, le cyclopropyltriméthoxysilane, le cyclobutyltriméthoxysilane, le cyclopentyltriméthoxysilane, le cyclohexyltriméthoxysilane, le cycloheptyltriméthoxysilane et le cyclooctyltriméthoxysilane.

7. Nanoparticule de silice mésoporeuse selon la revendication 1, dans laquelle la quantité de fraction hydrocarbyle terminale par particule est inférieure à 1 × 10⁶ molécule/particule.

8. Nanoparticule de silice mésoporeuse selon la revendication 1, dans laquelle elle comprend en outre au moins un ingrédient bioactif hydrophobe ou au moins un ingrédient bioactif hydrophile chargé dans les pores.

9. Nanoparticule de silice mésoporeuse selon la revendication 8, dans laquelle l'ingrédient bioactif hydrophobe est une petite molécule, un médicament de chimiothérapie, une enzyme, un médicament protéique, un anticorps, un vaccin, un antibiotique, un médicament nucléotide ou des combinaisons de ceux-ci.

10. Nanoparticule de silice mésoporeuse selon la revendication 8, dans laquelle elle comprend en outre au moins un ingrédient bioactif hydrophile ou hydrophobe chargé dans les pores.

11. Nanoparticule de silice mésoporeuse selon la revendication 10, dans laquelle les ingrédients bioactifs présentent un effet synergique.

12. Nanoparticule de silice mésoporeuse selon l'une quelconque des revendications 1 à 11, destinée à être utilisée dans un procédé pour délivrer un ou des ingrédients bioactifs à un sujet, comprenant :
(1) le chargement des ingrédients bioactifs dans les pores de la nanoparticule de silice mésoporeuse selon l'une quelconque des revendications 1 à 11 ; et
(2) l'administration de la nanoparticule de silice mésoporeuse décrite dans (1) au sujet.

13. Nanoparticule de silice mésoporeuse destinée à être utilisée dans un procédé selon la revendication 12, dans laquelle la nanoparticule de silice mésoporeuse est délivrée pour pénétrer une barrière cérébrale sanguine.

14. Nanoparticule de silice mésoporeuse destinée à être utilisée dans un procédé selon la revendication 12, dans laquelle la nanoparticule de silice mésoporeuse est délivrée pour pénétrer une barrière oculaire sanguine.

15. Procédé de préparation d'une nanoparticule de silice mésoporeuse (MSN) avec une modification organique de surface interne sur les pores, comprenant les étapes consistant à :
(a) fournir une solution alcaline contenant un tensioactif pour former des micelles ;
(b) ajouter une première source de silice et une seconde source de silice qui fournit une fraction hydrocarbyle terminale dans la solution, dans lequel le rapport molaire de la première source de silice et de la seconde source de silice n'est pas inférieur à 5:1 ;
(c) traiter hydrothermal de la solution ; et
(d) extraire et éventuellement purifier les MSN de la solution.

16. Procédé selon la revendication 15, dans lequel le procédé comprend en outre au moins l'une des étapes suivantes :
(e) l'introduction de phase huileuse dans la solution après l'étape (a) et avant l'étape (b) pour l'extension de poreuse ;
(f) l'ajout d'une source supplémentaire de silice après l'étape (b) ; et
(g) la réalisation d'une modification de surface de la surface externe des MSN, dans lequel la modification de surface est réalisée après l'étape (b), ou après l'étape (f) si l'étape (f) est réalisée.

17. Procédé selon la revendication 15, dans lequel l'agent tensioactif est un agent tensioactif cationique, un agent tensioactif anionique, un agent tensioactif non ionique ou toute combinaison de ceux-ci.

18. Procédé selon la revendication 15, dans lequel la première source de silice comprend du tétraéthoxysilane (TEOS), du tétraméthoxysilane (TMOS), du silicate de sodium ou un mélange de ceux-ci.

19. Procédé selon la revendication 15, dans lequel la deuxième source de silice est choisie dans le groupe constitué de triméthoxyphenylsilane (TMPS), triéthoxyphenylsilane (TEPS), diphenyldiéthoxysilane, 1-naphthyl triméthoxysilane, 2-hydroxy-4-(3-triéthoxy silylpropoxy)diphenylketone, O-4-méthylcoumarinyl-N-[3-(triéthoxysilyl)propyl]carbamate, 7-triéthoxysilylpropoxy-5-hydroxyflavone, 3-carbazolylpropyltriéthoxysilane, bis(2-diphenylphosphinoéthyl)méthylsilyléthyltriéthoxysilane, 2-(diphenylphosphino)éthyl triéthoxysilane, propyltriéthoxysilane n-butyltriéthoxysilane, pentyltriéthoxysilane, hexyltriéthoxysilane, heptyltriéthoxysilane, octyltriéthoxysilane, nonyltriéthoxysilane, décyltriéthoxysilane, undécyltriethoxysilane, dodécyltriéthoxysilane, cyclopropyltriéthoxysilane, cyclobutyltriéthoxysilane, cyclopentyltriéthoxysilane, cyclohexyltriéthoxysilane, cycloheptyltriéthoxysilane, cyclooctyltriéthoxysilane, propyltriméthoxysilane, n-butyltriméthoxysilane, pentyltriméthoxysilane. hexyltriméthoxysilane, heptyltriméthoxysilane, octyltriméthoxysilane, nonyltriméthoxysilane, décyltriméthoxysilane, undécyltriméthoxysilane, dodécyltriméthoxysilane, cyclopropyltriméthoxysilane, cyclobutyltriméthoxysilane, cyclopentyltriméthoxysilane, cyclohexyltriméthoxysilane, cycloheptyltriméthoxysilane et cyclooctyltriméthoxysilane.

20. Procédé selon la revendication 15, dans lequel la phase huileuse décrite dans l'étape (e) comprend un ou des (cyclo)alcanes substitués ou non substitués, un ou des solvants aromatiques substitués ou non substitués ou des combinaisons de ceux-ci.

21. Nanoparticule de silice mésoporeuse, qui est préparée par le procédé selon l'une quelconque des revendications 15 à 20.
